# EUROPÄISCHE PATENTSCHRIFT

(11) **EP 3 374 018 B1**
(45) Veröffentlichungstag und Bekanntmachung des Hinweises auf die Patenterteilung: **18.08.2021**
(21) Anmeldenummer: 16798403.8
(22) Anmeldetag: 11.11.2016
(51) Int. Cl.: A61M 5/168, A61M 1/36, A61M 39/28

(54) **SCHAUCHKLEMME FÜR EIN BLUTBEHANDLUNGSGERÄT**
HOSE CLAMP FOR A BLOOD TREATMENT DEVICE
CLAMP POUR TUYAU FLEXIBLE POUR UN APPAREIL DE TRAITEMENT DU SANG

(30) Priorität: 13.11.2015 DE 102015014741
(43) Veröffentlichungstag der Anmeldung: 19.09.2018
(73) Patentinhaber: Fresenius Medical Care Deutschland GmbH, 61352 Bad Homburg (DE)
(72) Erfinder: BREHM, Winfried, 97461 Hofheim (DE); WIESEN, Gerhard, 61352 Bad Homburg (DE); FISCHER, Karsten, 97421 Schweinfurt (DE)
(74) Vertreter: Herrmann, Uwe
(86) Internationale Anmeldenummer: PCT/EP2016/001879
(87) Internationale Veröffentlichungsnummer: WO 2017/080663

(56) Entgegenhaltungen:
- CA-A1- 2 913 091
- US-A1- 2010 057 016

## Beschreibung

Die Erfindung betrifft eine Schlauchklemme für ein Blutbehandlungsgerät, ein Blutbehandlungsgerät mit einer derartigen Schlauchklemme sowie ein Verfahren zum Entnehmen oder Einlegen eines Schlauchsets aus einem bzw. in ein derartiges Blutbehandlungsgerät.

Als Sicherheitsfeature haben Blutbehandlungsgeräte eine Schlauchklemme, die zum Abklemmen eines Schlauchs des extrakorporalen Blutkreislaufs dient. Die Klemme ermöglicht, den Patienten im Verlauf der Behandlung vom extrakorporalen Blutkreislauf zu trennen, insbesondere um einen Blutverlust zu vermeiden. Aus Sicherheitsgründen ist es bekannt, die Klemme so auszulegen, dass sie im stromlosen Zustand, der beispielweise durch einen Stromausfall hervorgerufen werden kann, schließt und so den Patienten vom extrakorporalen Blutkreislauf trennt.

Im herkömmlichen Dreischichtbetrieb von Blutbehandlungszentren und beispielsweise Dialysezentren werden die Geräte nach der letzten Behandlung am Abend bereits für den Folgetag vorbereitet und der als extrakorporaler Blutkreislauf dienende Schlauchsatz wird eingelegt.

Da sich das Gerät über Nacht aber regelmäßig im stromlosen Zustand befindet, klemmt die Klemme regelmäßig über einen längeren Zeitraum den Schlauch. Dies führt zu einer dauerhaften Quetschung des Schlauchs, der dadurch in dem betroffenen Bereich irreversibel verformt wird. Dies kann zu unerwünschten Turbulenzen im Betrieb führen.

Beispielsweise offenbart die Druckschrift US2010/057016A1 eine Schlauchklemme, welche bei einem Stromausfall in der geschlossenen Position verbleibt. Ziel der Erfindung ist es, diesen Nachteil zu vermeiden.

Vor diesem Hintergrund betrifft die Erfindung eine Schlauchklemme für ein Blutbehandlungsgerät, die ein Gehäuse und ein elektromechanisch angetriebenes Druckstück aufweist. Der elektromechanische Antrieb des Druckstücks ist derart ausgebildet, dass es in einem bestromten Zustand der Schlauchklemme gegen eine mechanische Rückstellkraft geöffnet wird und in einem stromlosen Zustand der Schlauchklemme durch die mechanische Rückstellkraft geschlossen wird. Zusätzlich zum elektromechanischen Antrieb weist die Schlauchklemme einen mechanischen Aktor auf, mit dem das Druckstück im stromlosen Zustand der Schlauchklemme manuell gegen die mechanische Rückstellkraft geöffnet werden kann. Erfindungsgemäß ist vorgesehen, dass die Kontaktelemente des Aktors und des Druckstücks so ausgebildet sind, dass sie nach einem Öffnen des Druckstücks im stromlosen Zustand eine mechanische Verbindung zwischen dem Aktor und dem Druckstück ausbilden, die ein erneutes Schließen des Druckstücks im stromlosen Zustand der Schlauchklemme durch die mechanische Rückstellkraft verhindert, und die im bestromten Zustand der Schlauchklemme gelöst wird.

Es handelt sich bei einem Kontaktelement, vorzugsweise bei dem druckstückseitigen Kontaktelement um einen Vorsprung und bei dem anderen Kontaktelement, vorzugsweise bei dem aktorseitigen Kontaktelement um eine Führungsbahn. Die Führungsbahn ist dabei so ausgebildet, dass sich der Vorsprung bei Betätigung des Aktors daran entlang bewegt. Ferner umfasst die Führungsbahn an einem Sattelpunkt, an dem sich der Vorsprung in einer geöffneten Position des Druckstücks befindet, eine Rastkontur für den Vorsprung. Bei der Führungsbahn kann es sich beispielsweise um eine Kante des Aktors oder um eine Kulissenführung im Aktor oder im Druckstück handeln.

Es handelt sich bei dem Aktor um einen verschwenkbaren Hebel, wobei vorzugsweise die Führungsbahn an einer Seitenkante des Hebelarms angeordnet ist.

In einer Ausführungsform ist die Führungsbahn zumindest abschnittsweise konvex und vorzugsweise teilkreisförmig ausgebildet.

In einer Ausführungsform befindet sich am Ende der Führungsbahn eine Stoppkontur. Vorzugsweise befindet sich die Stoppkontur jenseits des Sattelpunktes. Die Stoppkontur begrenzt die maximal durch den Aktor zu erreichende Öffnung des Druckstücks. Beispielsweise kann es sich bei der Stoppkontur um einen linearen Abschnitt der Führungsbahn handeln, der eine andere Steigung aufweist, als die vor dem Sattelpunkt liegenden Abschnitte.

In einer Ausführungsform handelt es sich bei dem Vorsprung um einen Stift, der vorzugsweise normal zur Schließrichtung des Druckstücks steht. Der Stift kann beispielsweise drehbar am Druckstück oder am Aktor gelagert sein, sodass er entlang der Führungsbahn abrollt oder fest gelagert sein, sodass er entlang der Führungsbahn gleitet.

In einer Ausführungsform handelt es sich bei der Rastkontur um eine Vertiefung in der Führungsbahn und/oder um eine Stufe auf der Führungsbahn. Die Vertiefung kann beispielsweise eine kreisförmige Einformung in der Führungsbahn am Sattelpunkt sein, wobei der Radius der kreisförmigen Einformung beispielsweise dem Radius des Stiftes entsprechen kann.

In einer Ausführungsform weist die Schlauchklemme eine mit dem Aktor und dem Gehäuse verbundene mechanische Feder, vorzugsweise eine mechanische Zugfeder auf, die eine Rückstellkraft auf den Aktor gegen dessen Betätigungsrichtung beim Öffnen des Druckstücks ausübt.

Vor dem eingangs genannten Hintergrund betrifft die Erfindung ferner ein Blutbehandlungsgerät, vorzugsweise ein Dialysegerät mit einem extrakorporalen Blutkreislauf, der einen als Schlauch ausgebildeten Leitungsabschnitt aufweist, wobei der Leitungsabschnitt vorzugsweise einen Teil der arteriellen oder der venösen Leitung des extrakorporalen Blutkreislaufs ausbildet. Erfindungsgemäß ist vorgesehen, dass an dem als Schlauch ausgebildeten Leitungsabschnitt eine erfindungsgemäße Schlauchklemme angeordnet ist.

Bei dem Dialysegerät kann es sich beispielsweise um ein Gerät zur Durchführung einer Hämodialyse, Hämodiafiltration und/oder Hämofiltration handeln.

Bei dem Schlauch handelt es sich vorzugsweise um einen Schlauch aus Kunststoff, der beispielsweise einen runden Querschnitt haben kann.

Die arterielle Leitung liegt in Strömungsrichtung vor und die venöse Leitung nach der Behandlungseinrichtung des Gerätes, beispielsweise dem Dialysator des Dialysegeräts. Je nach Lage des Leitungsabschnitts handelt es sich bei der daran angeordneten Klemme um eine arterielle oder eine venöse Klemme. Vorzugsweise liegen der Leitungsabschnitt und die Klemme nahe des arteriellen oder venösen Ports, an dem der Patient an den Blutkreislauf angeschlossen ist.

Der extrakorporale Blutkreislauf kann selbstverständlich mehrere als Schlauch ausgebildete Leitungsabschnitte aufweisen und das Blutbehandlungsgerät kann selbstverständlich mehrere gleich oder unterschiedlich ausgebildete Klemmen aufweisen, die an einem oder mehreren dieser Leitungsabschnitte eingreifen. Erfindungswesentlich ist, dass zumindest ein als Schlauch ausgebildeter Leitungsabschnitt vorhanden ist, an dem zumindest eine erfindungsgemäße Klemme angeordnet ist.

In einer Ausführungsform weist der extrakorporale Blutkreislauf zwei als Schläuche ausgebildete Leitungsabschnitte auf, wobei einer dieser Leitungsabschnitte einen Teil der arteriellen und der andere dieser Leitungsabschnitt einen Teil der venösen Leitung des extrakorporalen Blutkreislaufs ausbildet. In dieser Ausführungsform kann vorgesehen sein, dass das Blutbehandlungsgerät zwei erfindungsgemäße Schlauchklemmen aufweist, wobei eine dieser Schlauchklemmen an einem dieser Leitungsabschnitte und die andere der Schlauchklemmen an dem anderen dieser Leitungsabschnitte angeordnet ist.

Des Weiteren betriff die Erfindung vor dem eingangs genannten Hintergrund ein Verfahren zum Entnehmen oder Einlegen eines Schlauchsets aus einem bzw. in ein erfindungsgemäßes Blutbehandlungsgerät. Das Schlauchset bildet wenigstens einen Teil des extrakorporalen Blutkreislaufs aus und umfasst den Leitungsabschnitt, an dem die erfindungsgemäße Schlauchklemme angeordnet ist. Erfindungsgemäß ist vorgesehen, dass das Druckstück im stromlosen Zustand durch Betätigung des Aktors geöffnet wird und das Schlauchset anschließend im weiterhin stromlosen Zustand der Schlauchklemme entnommen oder eingelegt wird, während das Druckstück durch die mechanische Verbindung zwischen dem Aktor und dem Druckstück offen gehalten wird.

Das Entnehmen und Einlegen des Schlauchsets steht zusammengenommen für den Austausch des Schlauchsets.

Auf diese Weise kann beispielsweise in einem Dialysezentrum das Schlauchset bereits am Vorabend ausgetauscht bzw. eingelegt werden und die Dialysemaschine kann dennoch über Nacht stromlos bleiben, ohne dass das Schlauchset geklemmt und dabei ggf. verformt oder beschädigt wird.

Weitere Einzelheiten und Vorteile der Erfindung ergeben sich aus den nachfolgend anhand der Figuren erklärten bekannten und erfindungsgemäßen Systemen. In den Figuren zeigen:
- Figur 1:: eine Schnittdarstellung einer Schlauchklemme aus dem Stand der Technik in ihrem stromlosen und geschlossenen Zustand;
- Figur 2:: eine Schnittdarstellungen der in Figur 1 gezeigten Schlauchklemme im weiterhin stromlosen aber manuell geöffneten Zustand; und
- Figur 3:: eine Schnittdarstellung einer erfindungsgemäßen Schlauchklemme im stromlosen aber manuell geöffneten Zustand.

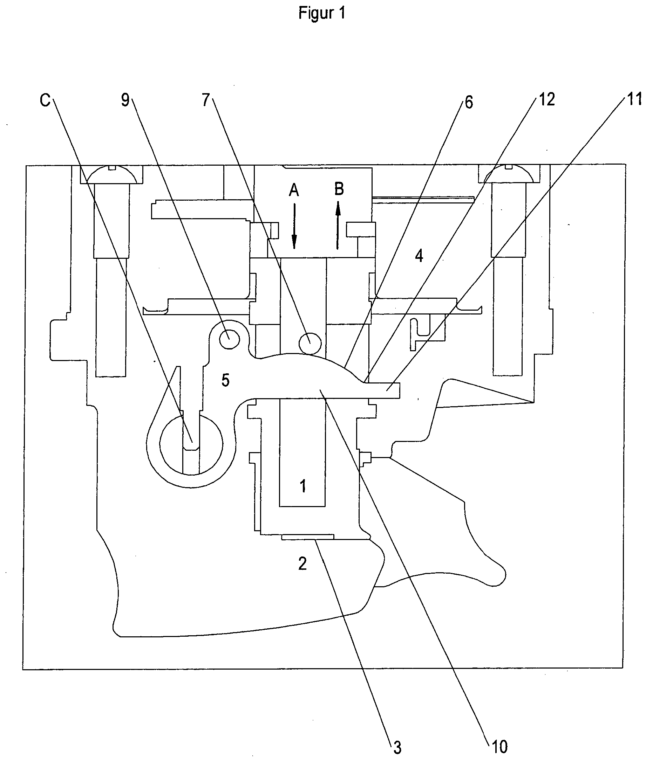

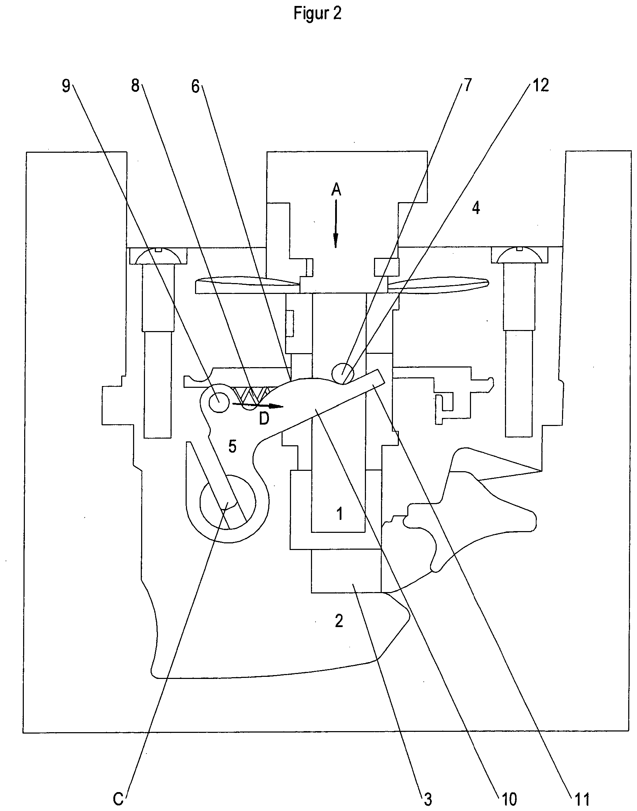

Figuren 1 und 2 zeigen eine vorbekannte Schlauchklemme eines Dialysegerätes aus dem Stand der Technik.

Die Schlauchklemme umfasst ein bewegliches Druckstück 1 und einen ortsfesten Sattel 2. Dazwischen ist eine Ausnehmung 3 zur Aufnahme eines Kunststoffschlauchs ausgebildet, beispielsweise eines Kunststoffschlauchs, der einen Teilabschnitt eines extrakorporalen Blutkreislaufes eines Dialysegerätes bildet.

Das Druckstück 1 ist innerhalb des Klemmengehäuses 4 vertikalverschieblich gelagert (Richtungen bzw. Positionen wie *"vertikal", "horizontal", "oben", "unten"* etc. beziehen sich auf die gezeigte Darstellung und nicht auf die tatsächliche Einbaulage der Schlauchklemme im Blutbehandlungsgerät). Die Vertikalverschiebung des Druckstücks wird anhand einer in den Figuren nicht näher dargestellten Elektromechanik gesteuert. Diese Elektromechanik umfasst einerseits eine mechanische Druckfeder, welche das Druckstück 1 nach unten (Richtung A) in die in Figur 1 dargestellte geschlossene Stellung drückt. Ferner umfasst die Elektromechanik einen Elektromagneten, der das Druckstück 1 im bestromten Zustand gegen die Rückstellkraft der mechanischen Druckfeder nach oben (Richtung B) zieht. Die vom Elektromagneten bewirkte öffnende Kraft ist größer als die von der mechanischen Druckfeder bewirkte schließende Kraft. Der durch Bestromung des Elektromagneten geöffnete Zustand der Klemme ist in den Figuren nicht dargestellt.

Neben dem Anheben des Druckstücks 1 durch Bestromung des Elektromagneten ist ferner ein manuelles Anheben des Druckstücks 1 durch Betätigung eines verschwenkbaren Hebels 5 möglich. Der Hebel 5 greift durch einen Durchbruch des Druckstücks 1 hindurch und umfasst eine Führungsbahn 6, welche mit einem Stift 7 zusammenwirkt, der am Druckstück 1 angeformt oder befestigt ist. Der Hebel 5 kann durch manuelle Betätigung um eine Achse C unter Überwindung einer mechanischen Gegenkraft aus einer horizontalen Ruheposition (Figur 1) in eine angehobene Position (Figur 2) geschwenkt werden. Die mechanische Gegenkraft wird dabei zum einen durch die Zugfeder 8 ausgeübt, welche zwischen dem Klemmengehäuse 4 und einem Fortsatz 9 des Hebels 5 angeordnet ist, und zum anderen durch die Rückstellkraft, die auf das Druckstück 1 wirkt und anhand der Kontaktelemente 6 und 7 an den Hebel 5 weitergegeben wird. Das in der Verlängerung der Achse C sitzende Betätigungselement zum manuellen Verschwenken des Hebels, bei dem es sich beispielsweise um einen Drehknopf oder um einen Griffhebel handeln kann, ist in den Figuren nicht näher dargestellt und wird vom Gehäuse 4 verdeckt.

Die Führungsbahn 6 umfasst eine konvexe Ausbauchung an der Oberseite des Hebelarmes 10 sowie einen geraden und parallel zur Richtung des Hebelarms verlaufenden Endbereich 11. Zwischen der konvexen Ausbauchung und dem geraden Abschnitt befindet sich der Sattelpunkt 12.

Im stromlosen Ausgangszustand der Schlauchklemme gemäß Figur 1 befindet sich der Hebel 5 in seinem Ruhezustand. Soll in diesem Zustand ein Schlauch aus der Aufnahme 3 entfernt werden oder ein Schlauch in die Aufnahme 3 eingelegt werden, so kann das Druckstück 1 durch Betätigung des Hebels angehoben werden. In der Ausgangsposition wird der Hebel 5 durch die Zugfeder 8 in horizontaler Position gehalten. Der Stift 7 des Druckstückes 1 liegt in etwa in der Mitte der konvexen Führungsbahn 6 auf. Wird ausgehend von dieser Position der Hebel 5 durch manuelle Betätigung nach oben geschwenkt, wird der auf der Führungsbahn 6 aufliegende Stift 7 und somit das Druckstück 1 entgegen der mechanischen Federkraft A angehoben. Die maximale Amplitude dieses mechanischen Anhebens bzw. Öffnens der Klemme ist erreicht, wenn der Stift am Sattelpunkt 12 des Hebels 5 angelangt ist und ein weiteres Anheben durch die abrupte Änderung der Steigung der Führungsbahn 6 nicht mehr möglich ist.

Durch die in Richtung D gerichtete Rückstellkraft der Zugfeder 8 und durch die vom Stift 7 über die Führungsbahn 6 an den Hebel 5 weitergegebene Rückstellkraft des Druckstücks 1 in Richtung A wird der Hebel 5 nach Lösen der manuellen Gegenkraft automatisch in seine horizontale Ausgangsposition zurückverschwenkt.

Auf das mechanische Schließen der Klemme im stromlosen Zustand hat der Hebel 5 also wenn er nicht gerade betätigt wird keinen Einfluss.

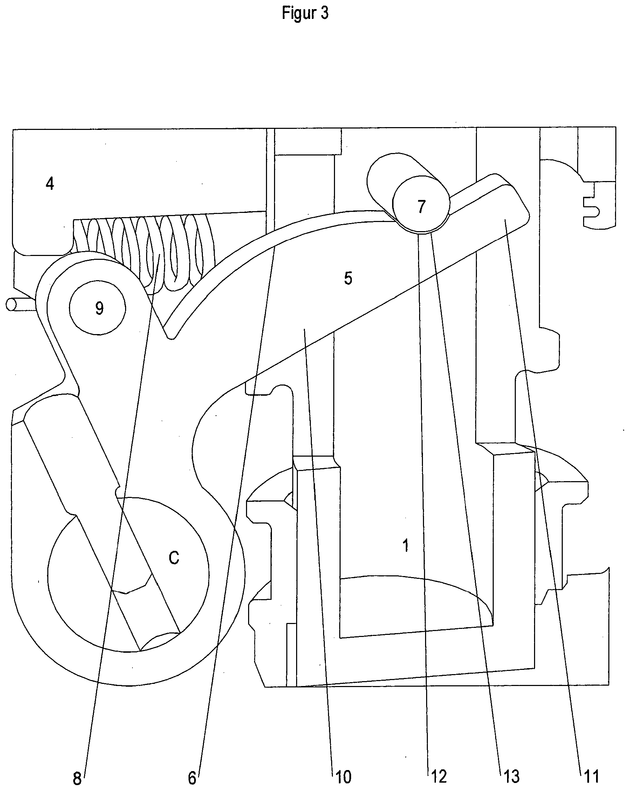

Figur 3 zeigt eine Schnittdarstellung einer erfindungsgemäßen Schlauchklemme im stromlosen und manuell geöffneten Zustand, wie dieser in Figur 2 für die Schlauchklemme aus dem Stand der Technik gezeigt ist.

Im Gegensatz zu der Schlauchklemme aus dem Stand der Technik ist erfindungsgemäß am Sattelpunkt 12 des Hebels 5 eine Rastkontur in Form einer Vertiefung 13 in der Führungsbahn 6 angeordnet. Die Vertiefung 13 ist in der gezeigten Ausführungsform teilkreisförmig, wobei der Radius des Teilkreises dem Außenradius des Stiftes 7 entspricht.

Wenn der Stift 7 bei einem manuellen Anheben des Druckstückes 1 solange an der Führungsbahn entlanggelaufen ist, bis der Sattelpunkt 12 erreicht ist, senkt sich der Stift 7 in dieser Vertiefung. Dadurch wird ein Rücklaufen des Stiftes 7 entlang der Führungsbahn 6 verhindert, da die in Richtungen A und D wirkenden Kräfte den Stift 7 gegen die Seitenfläche der Vertiefung 13 drücken, was zu einer Verkeilung bzw. Verrastung des Druckstücks 1 mit dem Hebel 5 führt. Dies verhindert ein Rückschwenken des Hebels 5 in seine horizontale Ruheposition und ein Absenken des Druckstückes 1 in Richtung A.

Selbstverständlich sind neben der gezeigten Einformung 13 auch andere Rastmittel wie beispielsweise eine Stufe an der Führungsbahn 6 denkbar.

Durch die Rastkontur bleibt also zusammenfassend die Schlauchklemme nach einem mechanischen Öffnen der Klemme im stromlosen Zustand geöffnet.

Diese durch die Rastkontur 13 hervorgerufene Blockade wird bei einem Bestromen der Klemme automatisch und ohne weiteres manuelles Zutun gelöst. Denn bei einem Bestromen der Klemme wird das Druckstück 1 ausgehend von der in Figuren 3 gezeigten Position zusätzlich angehoben und die Schlauchklemme noch etwas weiter geöffnet. Durch das über das mechanische Anheben hinausgehende magnetische Anheben wird die Rastverbindung zwischen dem Stift 7 und der Rastkontur 13 gelöst, und der Hebel 5 wird durch die Zugkraft der Feder 8 in Richtung D in seine Ausgangsposition (horizontal) zurückgeschwenkt. Im Betrieb ist also die Funktion der Schlauchklemme gemäß der Erfindung identisch mit der Funktion der Klemme aus dem Stand der Technik.

Zusammenfassend besteht die erfinderische Idee also darin, die Schlauchklemme mit einer Halte- und insbesondere Rastfunktion zu versehen, die beispielsweise abends beispielsweise manuell aktiviert werden kann, so dass der Schlauch des extrakorporalen Blutkreislaufs trotz Stromlosigkeit über Nacht nicht geklemmt wird. Mit Start des Geräts am nächsten Tag kann die Rastfunktion deaktiviert werden. Im Verlauf der Behandlung kann die Klemme dann den Schlauch klemmen, wenn das Dialysegerät stromlos ist, wie dies aus dem Stand der Technik bekannt ist.

## Patentansprüche

1. Schlauchklemme für ein Blutbehandlungsgerät, wobei die Schlauchklemme ein Gehäuse (4) und ein elektromechanisch angetriebenes Druckstück (1) aufweist, wobei
der elektromechanische Antrieb des Druckstücks (1) derart ausgebildet ist, dass das Druckstück (1) in einem bestromten Zustand der Schlauchklemme gegen eine mechanische Rückstellkraft (A) geöffnet wird und in einem stromlosen Zustand der Schlauchklemme durch die mechanische Rückstellkraft (A) geschlossen wird, und
die Schlauchklemme zusätzlich zum elektromechanischen Antrieb einen verschwenkbaren Hebel (5) aufweist, mit dem das Druckstück (1) im stromlosen Zustand der Schlauchklemme manuell gegen die mechanische Rückstellkraft (A) geöffnet werden kann,
**dadurch gekennzeichnet, dass**
die Kontaktelemente (6, 7) des Hebels (5) und des Druckstücks (1) ein Vorsprung (7) und eine Führungsbahn (6) sind, die so ausgebildet sind, dass sie nach einem Öffnen des Druckstücks (1) im stromlosen Zustand eine mechanische Verbindung zwischen dem Hebel (5) und dem Druckstück (1) ausbilden, wobei
die mechanische Verbindung eine Rastkontur (13) der Führungsbahn zum Verrasten des Vorsprungs (7) mit dem Hebel (5) aufweist, die ein erneutes Schließen des Druckstücks (1) im stromlosen Zustand der Schlauchklemme, bedingt durch die mechanische Rückstellkraft (A), verhindert und die im bestromten Zustand der Schlauchklemme gelöst wird, da das Druckstück (1) im bestromten Zustand zusätzlich angehoben wird, wodurch die Rastverbindung gelöst wird,
und wobei die Führungsbahn (6) an einem Sattelpunkt (12), an dem sich der Vorsprung (7) in einer geöffneten Position des Druckstücks (1) befindet, die Rastkontur (13) für den Vorsprung (7) aufweist.

2. Schlauchklemme nach Anspruch 1, wobei es sich bei dem druckstückseitigen Kontaktelement (7) um den Vorsprung (7) handelt und bei dem aktorseitigen Kontaktelement (6) um die Führungsbahn (6) handelt, wobei die Führungsbahn (6) so ausgebildet ist, dass sich der Vorsprung (7) bei Betätigung des Aktors (5) daran entlang bewegt.

3. Schlauchklemme nach einem der vorhergehenden Ansprüche, wobei die Führungsbahn (6) an einer Seitenkante des Hebelarms (10) angeordnet ist.

4. Schlauchklemme nach Anspruch 2 oder 3, wobei die Führungsbahn (6) zumindest abschnittsweise konvex und vorzugsweise teilkreisförmig ausgebildet ist.

5. Schlauchklemme nach einem der Ansprüche 2 bis 4, wobei es sich bei dem Vorsprung (7) um einen Stift (7) handelt, der vorzugsweise normal auf die Schließrichtung (A) des Druckstücks (1) steht.

6. Schlauchklemme nach einem der Ansprüche 2 bis 5, wobei es sich bei der Rastkontur (13) um eine Vertiefung (13) in der Führungsbahn (6) und/oder um eine Stufe auf der Führungsbahn (6) handelt.

7. Schlauchklemme nach einem der vorhergehenden Ansprüche, wobei die Schlauchklemme eine mit dem Hebel (5) und dem Gehäuse (4) verbundene mechanische Feder (8) und vorzugsweise Zugfeder (8) umfasst, die eine Rückstellkraft (D) auf den Hebel (5) gegen dessen Betätigungsrichtung beim Öffnen des Druckstücks (1) ausübt.

8. Blutbehandlungsgerät, vorzugsweise Dialysegerät mit einem extrakorporalen Blutkreislauf, der einen als Schlauch ausgebildeten Leitungsabschnitt aufweist, wobei
der Leitungsabschnitt vorzugsweise einen Teil der arteriellen oder der venösen Leitung des extrakorporalen Blutkreislaufs ausbildet, wobei an dem als Schlauch ausgebildeten Leitungsabschnitt eine Schlauchklemme gemäß einem der vorhergehenden Ansprüche angeordnet ist.

9. Blutbehandlungsgerät nach Anspruch 8, wobei der extrakorporale Blutkreislauf zwei als Schläuche ausgebildete Leitungsabschnitte aufweist, wobei einer dieser Leitungsabschnitte einen Teil der arteriellen und der andere dieser Leitungsabschnitt einen Teil der venösen Leitung des extrakorporalen Blutkreislaufs ausbildet, und dass das Blutbehandlungsgerät zwei Schlauchklemmen aufweist, wobei eine dieser Schlauchklemmen an einem dieser Leitungsabschnitte und die andere der Schlauchklemmen an dem anderen dieser Leitungsabschnitte angeordnet ist, wobei es sich bei beiden Schlauchklemmen um Schlauchklemmen nach einem der Ansprüche 1 bis 7 handelt.

10. Verfahren zum Entnehmen und/oder Einlegen eines Schlauchsets aus einem bzw. in ein Blutbehandlungsgerät nach einem der Ansprüche 8 oder 9, wobei das Schlauchset wenigstens einen Teil des extrakorporalen Blutkreislaufs ausbildet und den Leitungsabschnitt umfasst, an dem die Schlauchklemme gemäß einem der Ansprüche 1 bis 7 angeordnet ist,
**dadurch gekennzeichnet, dass**
das Druckstück (1) im stromlosen Zustand durch Betätigung des Aktors (5) geöffnet wird und das Schlauchset anschließend im weiterhin stromlosen Zustand der Schlauchklemme entnommen oder eingelegt wird, während das Druckstück (1) durch die mechanische Verbindung zwischen dem Aktor (5) und dem Druckstück (1) offen gehalten wird.

## Claims

1. A hose clamp for a blood treatment device, wherein the hose clamp has a housing (4) and an electromagnetically driven compression piece (1), wherein
the electromagnetic drive of the compression piece (1) is configured such that the compression piece (1) is opened against a mechanical restoring force (A) in a state of the hose clamp with an applied current and is closed by the mechanical restoring force (A) in a currentless state of the hose clamp; and
the hose clamp has, in addition to the electromechanical drive, a pivotable lever (5) by which the compression piece (1) can be opened manually against the mechanical restoring force (A) in the currentless state of the hose clamp,
**characterized in that**
the contact elements (6, 7) of the lever (5) and of the compression piece (1) are a projection (7) and a guideway (6) that are configured such that they form a mechanical connection between the lever (5) and the compression piece (1) after an opening of the compression piece (1) in the currentless state, with
the mechanical connection having a latch contour (7) and a guideway (6) for latching the projection (7) to the lever (5) that prevents another closing of the compression piece (1) in the currentless state of the hose clamp due to the mechanical restoring force (A) and that is released in the state of the hose clamp with an applied current. since the compression piece (1) is additionally raised in the state with an applied current, whereby the latch connection is released,
and with the guideway (6) having the latch contour (13) for the projection (7) at a saddle point (12) at which the projection (7) is located in an opened position of the compression piece (1).

2. A hose clamp in accordance with claim 1, wherein the contact element (7) at the compression piece side is the projection (7) and the contact element (6) at the actuator side, is the guideway (6), with the guideway (6) being configured such that the projection (7) moves along it on actuation of the actuator (5).

3. A hose clamp in accordance with one of the preceding claims, wherein the guideway (6) is arranged at a side edge of the lever arm (10).

4. A hose clamp in accordance with claim 2 or claim 3, wherein the guideway (6) is at least sectionally convex and preferably of part circle shape.

5. A hose clamp in accordance with one of the claims 2 to 4, wherein the projection (7) is a pin (7) that preferably stands normal to the closing direction (A) of the compression piece (1).

6. A hose clamp in accordance with one of the claims 2 to 5, wherein the latch contour (13) is a recess (13) in the guideway (6) and/or a step on the guideway (6).

7. A hose clamp in accordance with one of the preceding claims, wherein the hose clamp comprises a mechanical spring (8), and preferably a tension spring (8), that is connected to the lever (5) and to the housing (4) and that exerts a restoring force (D) on the lever (5) against its actuation direction on the opening of the compression piece (1).

8. A blood treatment device, preferably a dialysis device having an extracorporeal blood circuit, that has a line section formed as a hose, wherein
the line section preferably forms a part of the arterial line or of the venous line of the extracorporeal blood circuit, and wherein
a hose clamp in accordance with one of the preceding claims is arranged at the line section formed as a hose.

9. A blood treatment device in accordance with claim 8, wherein the extracorporeal blood circuit has two line sections formed as hoses, with one of these line sections forming a part of the arterial line and the other of these line sections forming a part of the venous line of the extracorporeal blood circuit; and wherein the blood treatment device has two hose clamps, with one of these hose clamps being arranged at one of these line sections and the other one of the hose clamps being arranged at the other one of these line sections, with the two hose clamps being hose clamps in accordance with one of the claims 1 to 7.

10. A method of removing and/or inserting a hose kit out of or into a blood treatment device in accordance with one of the claims 8 or 9, wherein the hose kit forms at least a part of the extracorporeal blood circuit and comprises the line section at which the hose clamp in accordance with one of the claims 1 to 7 is arranged,
**characterized in that**
the compression piece (1) is opened in the currentless state by actuation of the actuator (5) and the hose kit is subsequently removed or inserted in the still currentless state of the hose clamp, while the compression piece (1) is kept open by the mechanical connection between the actuator (5) and the compression piece (1).

## Revendications

1. Clamp pour tuyau flexible pour un appareil de traitement du sang, dans lequel le clamp pour tuyau flexible présente un boîtier (4) et une pièce de pression (1) entraînée de manière électromécanique, dans lequel
l'entraînement électromécanique de la pièce de pression (1) est réalisé de telle manière que la pièce de pression (1) est ouverte dans un état alimenté en courant du clamp pour tuyau flexible à l'encontre d'une force de rappel (A) mécanique et est fermée dans un état sans courant du clamp pour tuyau flexible par la force de rappel (A) mécanique, et
le clamp pour tuyau flexible présente en plus de l'entraînement électromécanique un levier (5) pouvant pivoter, avec lequel la pièce de pression (1) peut être ouverte manuellement à l'encontre de la force de rappel (A) mécanique dans l'état sans courant du clamp pour tuyau flexible,
**caractérisé en ce que**
les éléments de contact (6, 7) du levier (5) et de la pièce de pression (1) sont une partie faisant saillie (7) et une voie de guidage (6) qui sont réalisées de telle sorte qu'elles réalisent, après une ouverture de la pièce de pression (1), dans l'état sans courant, une liaison mécanique entre le levier (5) et la pièce de pression (1), dans lequel
la liaison mécanique présente un contour d'enclenchement (13) de la voie de guidage pour enclencher la partie faisant saillie (7) avec le levier (5), qui empêche une nouvelle fermeture de la pièce de pression (1) dans l'état sans courant du clamp pour tuyau flexible, du fait de la force de rappel (A) mécanique et qui est desserré dans l'état alimenté en courant du clamp pour tuyau flexible dans la mesure où la pièce de pression (1) est relevée en supplément dans l'état alimenté en courant, ce qui permet de desserrer la liaison par enclenchement,
et dans lequel la voie de guidage (6) présente, sur un point de col (12), sur lequel se trouve la partie faisant saillie (7) dans une position ouverte de la pièce de pression (1), le contour d'enclenchement (13) pour la partie faisant saillie (7).

2. Clamp pour tuyau flexible selon la revendication 1, dans lequel l'élément de contact côté pièce de pression (7) est la partie faisant saillie (7) et l'élément de contact côté actionneur (6) est la voie de guidage (6), dans lequel la voie de guidage (6) est réalisée de telle sorte que la partie faisant saillie (7) se déplace lors de l'actionnement de l'actionneur (5) le long de celui-ci.

3. Clamp pour tuyau flexible selon l'une quelconque des revendications précédentes, dans lequel la voie de guidage (6) est disposée sur une arête latérale du bras de levier (10).

4. Clamp pour tuyau flexible selon la revendication 2 ou 3, dans lequel la voie de guidage (6) est réalisée au moins par endroits de manière convexe et de préférence en forme de cercle partiel.

5. Clamp pour tuyau flexible selon l'une quelconque des revendications 2 à 4, dans lequel la partie faisant saillie (7) est une tige (7), qui se dresse de préférence de manière normale sur la direction de fermeture (A) de la pièce de pression (1).

6. Clamp pour tuyau flexible selon l'une quelconque des revendications 2 à 5, dans lequel le contour d'enclenchement (13) est un renfoncement (13) dans la voie de guidage (6) et/ou un palier sur la voie de guidage (6).

7. Clamp pour tuyau flexible selon l'une quelconque des revendications précédentes, dans lequel le clamp pour tuyau flexible comprend un ressort (8) mécanique relié au levier (5) et au boîtier (4) et de préférence un ressort de traction (8), qui exerce une force de rappel (D) sur le levier (5) à l'encontre de son sens d'actionnement lors de l'ouverture de la pièce de pression (1).

8. Appareil de traitement du sang, de préférence appareil de dialyse avec un circuit extracorporel de sang, qui présente un tronçon de conduit réalisé en tant que tuyau flexible, dans lequel
le tronçon de conduit réalise de préférence une partie du conduit artériel ou du conduit veineux du circuit extracorporel de sang,
dans lequel un clamp pour tuyau flexible selon l'une quelconque des revendications précédentes est disposé sur le tronçon de conduit réalisé en tant que tuyau flexible.

9. Appareil de traitement du sang selon la revendication 8, dans lequel le circuit extracorporel de sang présente deux tronçons de conduit réalisés en tant que tuyaux flexibles, dans lequel un desdits tronçons de conduit réalise une partie du conduit artériel et l'autre desdits tronçons de conduit réalise une partie du conduit veineux du circuit extracorporel de sang, et l'appareil de traitement du sang présente deux clamps pour tuyau flexible, dans lequel un desdits clamps pour tuyau flexible est disposé sur un desdits tronçons de conduit et l'autre des clamps pour tuyau flexible est disposé sur l'autre desdits tronçons de conduit, dans lequel les deux clamps pour tuyau flexible sont des clamps pour tuyau flexible selon l'une quelconque des revendications 1 à 7.

10. Procédé pour retirer et/ou insérer un ensemble de tuyaux flexibles hors ou dans un appareil de traitement du sang selon l'une quelconque des revendications 8 ou 9, dans lequel l'ensemble de tuyaux flexibles réalise au moins une partie du circuit extracorporel de sang et comprend le tronçon de conduit, sur lequel le clamp pour tuyau flexible est disposé selon l'une quelconque des revendications 1 à 7,
**caractérisé en ce que**
la pièce de pression (1) est ouverte dans l'état sans courant par l'actionnement de l'actionneur (5) et l'ensemble de tuyaux flexibles est retiré ou inséré ensuite dans l'état par ailleurs sans courant du clamp pour tuyau flexible, tandis que la pièce de pression (1) est maintenue ouverte par la liaison mécanique entre l'actionneur (5) et la pièce de pression (1).
